# EUROPEAN PATENT SPECIFICATION

(11) **EP 0 854 701 B1**
(45) Date of publication and mention of the grant of the patent: **31.07.2002**
(21) Application number: 96900043.9
(22) Date of filing: 08.01.1996
(51) Int. Cl.: A61K 7/155, A45D 26/00

(54) **DEPILATORY STRIPS**
ENTHAARUNGSSTREIFEN
BANDES EPILATOIRES

(30) Priority: 09.01.1995 FR 9500156
(43) Date of publication of application: 29.07.1998
(73) Proprietor: Reckitt Benckiser France, 91301 Massy Cédex (FR)
(72) Inventor: PAILLE, Eric, F-28300 Gasville-Oiseme (FR); MARTIN, Georges, F-86280 Saint Benoit (FR)
(74) Representative: Dickson, Elizabeth Anne
(86) International application number: GB9600017
(87) International publication number: WO9621419

(56) References cited:
- FR-A- 1 390 484
- US-A- 3 808 637

## Description

The present invention relates to new depilatory strips and their application to depilation.

Depilation, particularly for cosmetic purposes, is performed by several techniques. The technique of electrolysis, electrical devices gripping and pulling out the hair, for example with the aid of rotating parts, chemical agents such as sulphides, the conventional tweezers, as well as hot and cold depilatory waxes, may be cited, for example.

Cold depilatory waxes are generally applied to a thin backing in the form of a strip. Such a wax will hereinafter be termed adhesive paste medium.

Traditionally, a film of cellophane comprising a coating of polyvinyl chloride on both sides is used by way of backing for the adhesive paste medium. The adhesive paste medium is deposited directly on one of the two sides coated with polyvinyl chloride. The quality of adhesion obtained between the adhesive paste medium and the backing is therefore the result of the adhesiveness between the adhesive paste medium and the polyvinyl chloride.

In addition to this first function as an adhesive surface for the adhesive paste medium, the polyvinyl chloride also acts as a barrier to prevent this adhesive paste medium from migrating through the backing film. In fact the consequences of the migration of the adhesive paste medium may be in particular the risks of undesirable bonding of the outer side of the strip, that is to say of the side opposite that on which the adhesive paste medium is deposited, or else reduced effectiveness of depilation.

Such cold wax strips are used as follows: the user presses the strip firmly onto the area to be depilated, the adhesive medium being placed in the direction of the area to be depilated, then one end of the backing strip is grasped and pulled sharply away from the area to be depilated.

The hairs trapped in the adhesive paste medium are therefore removed from the treated area and in principle the whole of the adhesive paste medium remains attached to the backing strip.

However, quite frequently residues of adhesive paste medium remain on the depilated area, and these very sticky residues are difficult to remove from the skin, particularly as the skin is often slightly irritated.

It would be desirable to have depilatory strips which would leave substantially no residues stuck to the treated skin.

In US-A-3,808,637 there is described a depilation device comprising a flexible carrier sheet material, suitably of polyethylene, having adhesively secured to it a layer of cellular material such as foamed polyurethane. The cellular material is impregnated with syrupy, tacky material.

The present invention relates to a depilatory strip comprising an adhesive paste medium deposited on a backing film, characterised in that one side of the backing film of single-layer or multi-layer plastics material is joined to a non-woven fabric on which the adhesive paste is deposited.

"Joined" means that the non-woven material is securely attached to the backing film for example by the techniques of extrusion coating or extrusion lamination.

By way of non-woven fabric, any non-woven fabric may be used, for example polypropylene-based, particularly calendered, or particularly polyester-based, having a gram weight of between 10 and 100 g per m². However, non-woven fabrics having a low gram weight, of between 10 and 40 g/m² for example are preferred, this in order to provide a better contact with the zone to be depilated, by virtue of the suppleness of the backing.

Depending on the area to be depilated and on the size of the strip, the person skilled in the art will have no difficulty in selecting the quality of the non-woven fabric to use.

The film of plastics material joined to the non-woven fabric may be for example a single-layer film or a multi-layer film, composed mainly of ethylene copolymer or ethylene glycol-vinyl alcohol copolymer whether or not coextruded with a polyethylene film, of non-orientated, mono-orientated or bi-orientated extruded polyamide in particular, of polyester, of polyethylene, of polypropylene, of bi-orientated polypropylene or of polyvinyl chloride for example. This film provides the barrier properties necessary in order to prevent any risk of bonding between the strips, which would be caused by the diffusion through the backing film of the adhesive paste medium or of one of its constituents. Films constituted by polyamide, by polyvinyl chloride or by an ethylene or ethylene glycol-vinyl alcohol copolymer are preferred.

Its thickness may range for example from 5 to 50 µm, and preferably from 10 to 30 µm.

As in the state of the art, the adhesive paste medium may be composed of 97% colophony resin and derivatives, with the addition of one or more colourings and perfumes.

Some commercial backing films joined to a non-woven fabric are for example those marketed by the PWA-K DUFFEL Company under the references 40 766 (non-woven polyester fabric 30 g/m² + ethylene-vinyl acetate copolymer 55 g/m²) or 41 516 (non-woven polyester fabric 17 g/m² + low density polyethylene 15 g/m² + polyamide 25 µm + low density polyethylene 30 g/m²) respectively.

Those marketed by the A.C.E. Company in particular under the reference LAMINE CODE 300 (calendered non-woven polypropylene fabric 40 g/m² + polyethylene 32 µm) may also be cited.

The outer face of the backing film may be treated to accept printing and/or an additional layer of a polymer to make it softer to the touch. The corona treatment may be cited for example.

The inner side of the film of plastics material, that is to say the non-woven side or the non-woven fabric, can accept a layer of an agent well known in the state of the art serving to provide a welding termed "peelable" of two strips relative to one another.

By way of agents serving to provide a weld termed "peelable" in order to allow easy separation of the two strips at the moment of use, extruded or laminated polyethylene may be cited for example, or a thermosealable lacquer heat-sealable by means of heating jaws for example; a coating cold-sealable by pressure, deposited by photogravure may further be cited. Polyethylene, whether peelable or not, is preferably retained.

Such an agent may be applied in the areas not covered with adhesive paste medium.

By placing two strips face to face, a strip is obtained double-welded on either side of the adhesive medium.

Such double strips have remarkable properties: running of the adhesive paste is thus prevented and these double strips retain their original clean and regular appearance in the event of their being subject for example to an abnormal rise in temperature during storage and/or transport.

During the use of these depilation strips, the two strips can be separated easily without damage to the backing and/or to the adhesive paste medium.

That is why the present application also relates to a depilatory strip characterised in that it is constituted by two single strips, as defined above, welded face to face by virtue of an agent permitting hot welding or cold welding to be carried out and providing mutual peelability between two strips, said agent being deposited on the areas of the backings not covered with adhesive paste medium, on the non-woven fabric side. Such an agent makes it possible, by virtue of its peelability properties, to separate the two parts of the depilatory strip easily and without damaging the backing film.

The depilatory strips according to the invention can be manufactured as follows:

In order to produce a single strip, adhesive paste medium is deposited on a continuous film (on a roll), by spraying hot and under pressure through a calibrated nozzle which determines the width of the adhesive medium. The timing of the spray and the rate of feed of the film determine the length and the quantity of adhesive medium deposited. The strip is then covered on the adhesive medium side with a protective film easy to remove such as a silicone coated paper, and then the whole is then cut to the length required in order to obtain a single strip.

In order to produce a double strip, the unitary strips being partially welded to one another, the same procedure as described above is followed except for the difference that the film is equivalent in width to two strips, and then that it is folded back onto itself after the adhesive medium has been deposited, without application of protective film. Welding if necessary by heating jaws, or by simple pressure in the case of a cold-sealable coating, takes place next, and then the cutting to the right length, as also the longitudinal cutting on the side of the part folded back, corresponding to the middle part of the original film, so as to obtain two separable strips.

A further possibility for depositing the adhesive medium for the production of double strips may be to use a film having a width equivalent to one strip and to transfer the adhesive medium by a process of the offset type onto two parallel strips fed in synchronisation. The width of the transfer roller determines the width of adhesive medium deposited and the angle of rotation of the roller determines the length of the adhesive paste medium deposited. The two strips are then placed face to face so as to obtain a separable double strip.

The present invention lastly relates to the application of a strip described above to depilation, as also a depilation process characterised in that a depilatory strip as defined above is used.

The invention will be better understood by reference to the accompanying drawings in which:

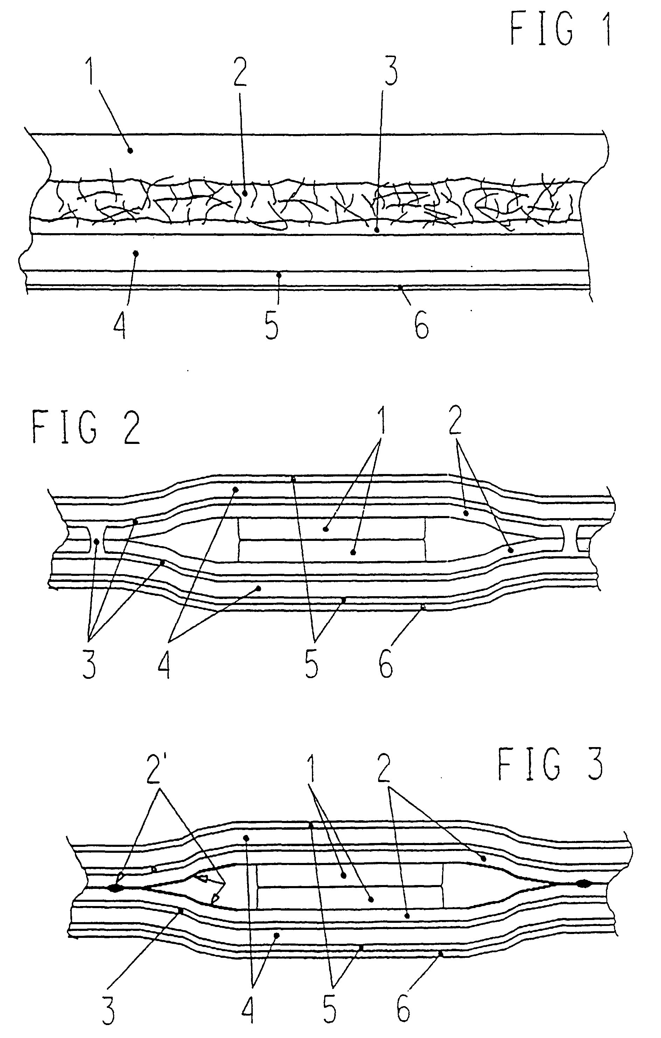

Figure 1 shows a single depilatory strip according to the invention, seen in cross-section, whereas Figures 2 and 3 show, also in cross-section, double depilatory strips according to the present invention.

In Figure 1, are seen, from top to bottom, the layer of adhesive paste medium 1, then the layer of non-woven fabric 2, next the bond layer 3 necessary to join the layer of non-woven fabric with the layer of barrier film 4. Lastly, and subsidiarily, there is an outer layer 5 protecting the barrier layer and making it possible to have a printed surface 6.

In Figure 2, which shows a double depilatory strip, consisting of two single strips disposed face to face as described above, and the bond layer 3 which here serves also as a layer for welding two strips to one another, the non-woven sides are seen to be placed face to face in this assembly.

In Figure 3, similar to Figure 2, a welding agent 2' is additionally seen which has been deposited directly onto the inner side of the non-woven fabric 2, in order to weld the two strips to one another.

### EXAMPLE 1 : Single depilatory strip

A depilatory strip was prepared having the following constitution with reference to Figure 1:
- perfumed adhesive paste medium, based on a colophony resin (1)
- backing film: - non-woven polyester fabric 20g/m² (2)
   - low density polyethylene 15 g/m² (3)
   - polyamide 15 µg (4)
   - low density polyethylene 15 g/m² (5)
   - printing on corona-treated surface (6)

### EXAMPLE 2 : Double depilatory strip

A depilatory strip was prepared having the following constitution with reference to Figures 2 and 3:
- perfumed adhesive paste medium, based on a colophony resin (1)
- backing film:
   - non-woven polyester fabric 20 g/m² (2)
   - thermosealable lacquer or varnish (2')
   - low density polyethylene 15 g/m² (3)
   - polyamide 15µg (4)
   - low density polyethylene 15 g/m² (5)
   - printing on corona-treated surface (6)

### EXAMPLE 3 Double depilatory strip

A depilatory strip was prepared having the following constitution with reference to Figures 2 and 3:
- perfumed adhesive paste medium, based on a colophony resin (1)
- backing film :
   - non-woven polyester fabric 40g/m² (2)
   - coating cold-sealable by simple pressure (a mixture of latex and acrylic marketed, for example, by the CRODA company)
   - low density polyethylene 15g/m² (3)
   - polyamide 15 µg (4)
   - low density polyethylene 15 g/m² (5)
   - printing on corona-treated surface (6)

## Claims

1. Depilatory strip comprising an adhesive paste medium (1) deposited on a backing film, **characterised in that** one side of the backing film (4) of single-layer or multi-layer plastics material is joined to a non-woven fabric (2) on which the adhesive paste medium (1) is deposited.

2. Depilatory strip according to Claim 1, **characterised in that** the gram weight of the non-woven fabric (2) is between 10 and 40 g/m².

3. Depilatory strip according to Claim 1 or 2, **characterised in that** the non-woven fabric (2) has a polyester or a calendered polypropylene base.

4. Depilatory strip according to Claim 1, 2 or 3, **characterised in that** the backing film has a thickness ranging from 10 to 30 µm.

5. Depilatory strip according to any of Claims 1 to 4, **characterised in that** the backing film is constituted by polyamide, by polyvinyl chloride, by ethylene-vinyl alcohol copolymer or by an ethylene glycol-vinyl alcohol copolymer.

6. Depilatory strip **characterised in that** it is constituted by two single strips as defined in Claim 1, welded face to face by virtue of an agent (2') making it possible to carry out hot or cold welding and providing peelability of two strips from one another, said agent (2') being deposited on the areas of the backings not covered by adhesive paste medium (1), on the non-woven fabric side (2).

7. Double depilatory strip according to Claim 6, **characterised in that** the welding agent (2') comprises polyethylene.

8. Application of a strip as defined in any of Claims 1 to 5 to cosmetic depilation.

9. Application of a strip as defined in any of claims 6 and 7 to cosmetic depilation.

## Patentansprüche

1. Depilations-Streifen, der ein auf einen Trägerfilm aufgebrachtes Klebstoffpasten-Medium (1) aufweist, **dadurch gekennzeichnet, dass** eine Seite des Trägerfilms (4) aus einem Einschichten- oder Mehrschichten-Kunststoffmaterial mit einem Vliesstoff (2) verbunden ist, auf den das Klebstoffpasten-Medium (1) aufgebracht ist.

2. Depilations-Streifen nach Anspruch 1, **dadurch gekennzeichnet, dass** das Flächengewicht des Vliesstoffes (2) zwischen 10 und 40 g/m² liegt.

3. Depilations-Streifen nach Anspruch 1 oder 2, **dadurch gekennzeichnet, dass** der Vliesstoff (2) eine Polyesteroder kalandrierte Polypropylen-Unterlage aufweist.

4. Depilations-Streifen nach Anspruch 1, 2 oder 3, **dadurch gekennzeichnet, dass** der Trägerfilm eine Dicke in dem Bereich von 10 bis 30 µm hat.

5. Depilations-Streifon nach einem der Ansprüche 1 bis 4, **dadurch gekennzeichnet, dass** der Trägerfilm besteht aus Polyamid, Polyvinylchlorid, einem Ethylen/Vinylalkohol-Copolymer oder einem Ethylenglycol/Vinylalkohol-Copolymer.

6. Depilations-Streifen, **dadurch gekennzeichnet, dass** er aus zwei Einzelstreifen, wie in Anspruch 1 definiert, besteht, die mittels eines Agens (2') flächenverschweißt sind, mit dessen Hilfe es möglich ist, eine Warm- oder Kaltverschweißung durchzuführen und die beiden Streifen voneinander abziehbar zu machen, wobei das Agens (2') auf die Bereiche der Träger, die nicht von dem Klebstoffpastenmedium (1) bedeckt sind, auf die Vliesstoff-Seite (2) aufgebracht ist.

7. Depilations-Doppelstreifen nach Anspruch 6, **dadurch gekennzeichnet, dass** das Verschweißungsmittel (2') Polyethylen umfasst.

8. Verwendung eines Streifens, wie er in einem der Ansprüche 1 bis 5 definiert ist, für die kosmetische Depilation (Haarentfernung).

9. Verwendung eines Streifens, wie er in einem der Ansprüche 6 und 7 definiert ist, für die kosmetische Depilation (Haarentfernung).

## Revendications

1. Bande épilatoire comprenant un milieu consistant en pâte adhésive (1) déposé sur un film de support, **caractérisée en ce qu'**une face du film de support (4) de matière plastique monocouche ou multicouche est jointe à une étoffe non tissée (2) sur laquelle est déposé le milieu consistant en pâte adhésive (1).

2. Bande épilatoire suivant la revendication 1, **caractérisée en ce que** le poids en grammes de l'étoffe non tissée (2) est compris dans l'intervalle de 10 à 40 g/m².

3. Bande épilatoire suivant la revendication 1 ou 2, **caractérisée en ce que** l'étoffé non tissée (2) a une base constituée d'un polyester ou d'un polypropylène calandré.

4. Bande épilatoire suivant la revendication 1, 2 ou 3, **caractérisée en ce que** le film de support a une épaisseur comprise dans l'intervalle de 10 à 30 µm.

5. Bande épilatoire suivant l'une quelconque des revendications 1 à 4, **caractérisée en ce que** le film de support est constitué d'un polyamide, d'un poly(chlorure de vinyle), d'un copolymère éthylène-alcool vinylique ou d'un copolymère éthylène-glycol-alcool vinylique.

6. Bande épilatoire, **caractérisée en ce qu'**elle est constituée de deux bandes distinctes répondant à la définition suivant la revendication 1, soudées face à face au moyen d'un agent (2') rendant possible d'effectuer un soudage à chaud ou à froid et permettant le pelage des deux bandes pour les séparer l'une de l'autre, ledit agent (2') étant déposé sur les zones des films de support non couvertes par le milieu consistant en pâte adhésive (1), sur la face d'étoffé non tissée (2).

7. Bande épilatoire double suivant la revendication 6, **caractérisée en ce que** l'agent de soudage (2') comprend le polyéthylène.

8. Application d'une bande répondant à la définition suivant l'une quelconque des revendications 1 à 5 à une épilation cosmétique.

9. Application d'une bande répondant à la définition suivant l'une quelconque des revendications 6 et 7 à une épilation cosmétique.
